(19) Europäisches Patentamt
European Patent Office
Office européen des brevets

(11) Publication number: **0 114 756**
**A2**

(12) # EUROPEAN PATENT APPLICATION

(21) Application number: 84300363.3

(22) Date of filing: 20.01.84

(51) Int. Cl.³: C 12 Q 1/24
C 12 N 9/18, C 12 N 5/00
C 12 N 11/04, A 61 K 37/54

(30) Priority: 21.01.83 US 459990

(43) Date of publication of application:
01.08.84 Bulletin 84/31

(84) Designated Contracting States:
CH DE FR GB IT LI

(71) Applicant: BIO-RESPONSE INC.
550 Ridgefield Road
Wilton Connecticut 06897(US)

(72) Inventor: Rose, Sam
2701 Claremont Boulevard
Berkeley California 94705(US)

(74) Representative: Marsh, Roy David et al,
Brewer & Son 5-9, Quality Court Chancery Lane
London WC2A 1HT(GB)

(54) **Isolation and culture of mammalian cells secreting acetylcholinesterase and recovery of acetylcholinesterase therefrom.**

(57) There is disclosed a method for isolating living mammalian cells secreting acetylcholinesterase (AChE) from a population of cells secreting AChE at varying rates, in which the cells of the population are encapsulated in a semipermeable layer (10) along with excess of a material which captures and immobilizes within the capsule all AChE secreted by the encapsulated cell, and then a product having an enzyme affixed to it is added to the population to bind only to the AChE or AChE complex i.e. in only those capsules containing an AChE-secreting cell. Thereafter, a substrate is added to the population for the purpose of establishing a physical difference between enzyme-containing and enzyme-free cells thereby permitting physical separation of these two categories of capsule. Subsequently the cells producing AChE can be recovered for culturing. The procedure can be performed again, on the recovered cells to isolate from them the cells with the highest rate of production of AChE.

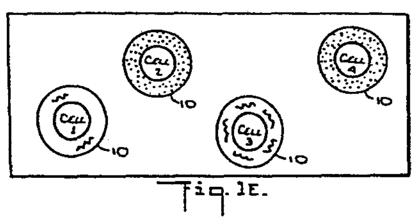


Fig. 1E.

EP 0 114 756 A2

# ISOLATION AND CULTURE OF MAMMALIAN CELLS SECRETING ACETYLCHOLINESTERASE AND RECOVERY OF ACETYLCHOLINESTERASE THEREFROM

The present invention relates to the production of acetylcholinesterase and, more particularly, to the isolation and *in vitro* culturing of living mammalian cells which secrete acetylcholinesterase, and the recovery of acetylcholinesterase therefrom.

Acetylcholinesterase (hereinafter "AChE") is a hydrolytic enzyme present in vertebrates and insects which catalyzes the hydrolysis of the ion acetylcholine.

Acetylcholine ion, represented by the formula

$$CH_3\overset{O}{C}OCH_2CH_2\overset{+}{N}(CH_3)_3,$$

is a neurotransmitter at synapses and at neuroeffector junctions of the cholinergic nervous system. Upon receipt of a nerve impulse, acetylcholine is liberated from storage vessels at cholinergic nerve endings and then interacts with a receptor on the membrane surrounding the next neuron (synapse), or the motor endplate, or the neuromuscular junction. This effects transmission of impulse by altering the permeability of the postjunctional membrane to permit the flow of ions necessary for excitation.

The receptor-acetylcholine complex rapidly dissociates and the liberated acetylcholine is rapidly hydrolyzed thereby terminating its effect in the impulse transmission process) in a reaction catalyzed by AChE, which is secreted by cells in the immediate vicinity of the acetylcholine-receptor interaction. This hydrolysis of acetylcholine proceeds at such an extremely rapid rate that the result is that the acetylcholine liberated by a single nerve impulse essentially transmits that, and only that, impulse to the effector cell. Were this not the case, the acetylcholine liberated by a single nerve impulse would bring about uncontrollable stimulation of the cholinergic nervous system, and discrete controlled nervous action would be impossible.

The inactivation of AChE (or the inhibition of its catalytic effect) in an otherwise normal subject therefore has the effect of preventing the rapid hydrolysis of acetylcholine, thereby causing unwanted, undesirable and uncontrollable nervous action; indeed, the inhibition of the hydrolysis of acetylcholine by AChE is the basis for the effectiveness of a number of insecticides, wherein AChE inhibitors (e.g., esters of carbamic acid or derivatives of the acids of phosphorus) are employed to debilitate the insects. The same principle underlies the formulation of nerve gases for use in chemical warfare. AChE inhibitors in the nerve gas (particularly phosphate derivatives) interfere with the AChE-catalyzed hydrolysis of acetylcholine and thereby bring about convulsive nervous action and eventual death.

The overt administration of acetylcholinesterase to a subject (e.g., a human) prior to the subject's exposure to an acetylcholinesterase inhibitor (e.g., a nerve gas)

affords a means for providing a prophylactic effect against the debilitating effects of the inhibitor on the cholinergic nervous system. The overt AChE administration (e.g., in a single dosage or in a periodic or continuous regimen) results in the presence of AChE in the circulatory system of the subject. This AChE has no physiological effect per se on the subject since its concentration is far below that of the AChE present on or near cells involved in the cholinergic nervous system. In other words, the overtly added AChE does not interfere with the normal functioning of the cholinergic nervous system.

When a subject treated in the foregoing manner is exposed to an AChE inhibitor, however, the AChE which was caused to be present in the circulatory system "intercepts" the inhibitor before the inhibitor can act upon the AChE involved in the functions of the subject's cholinergic nervous system. The overtly added ACnE presents to the inhibitor the precise material (AChE) upon which the inhibitor is designed to act and thereby ties up (or exhausts the inhibitory action of) the inhibitor. The effect of the inhibitor on the overtly added AChE is of no consequence since this AChE, even in its uninhibited state, performs no useful biological/physiological function; however, in this manner the inhibitor is prevented from reaching and acting (adversely) upon the AChE involved in the cholinergic nervous system functions.

The primary emphasis of the present invention involves the production of AChE in the substantial quantities which would be required in order to effectuate the above-described prophylactic treatment against AChE inhibitors, for example, where it may be desirable to administer AChE to a large number of military personnel as a counter to anticipated use by an enemy of nerve gas

or where it may be desired to administer AChE to persons involved in the manufacture, handling and distribution of AChE inhibitor-based nerve gases or insecticides.

AChE is a highly complex molecule and to date has not been synthetically prepared in any form which would be suitable for administration to a living subject and which would function in the manner in which AChE produced in vivo acts. So-called "recombinant DNA" technology does not offer a feasible method for producing AChE since the genes coded for AChE production have not to date been identified and isolated from mammalian cells.

AChE actually produced and secreted naturally by mammalian cells obviously would be the most desirable route to obtaining AChE in a form suitable for administration to a living subject for the prophylactic purposes earlier described. There are, in fact, a number of known cell lines which synthesize and secrete AChE in _in vitro_ culture, including embryonic cells (such as myoblasts from embryonic rat leg muscles, muscle cells from embryonic chicken pectoral muscles, chick myogenic cells from embryonic cells, chick ciliary ganglia cells from embryonic ciliary ganglia and neuronal cells from chick embryonic paravertebral sympathetic ganglia) and various types of transformed cells (e.g. Rat PC12 pheochromocytoma cells, mouse neuroblastoma cell lines and human neuroblastoma cell lines). However, the conditions under which such cells must be cultured in order to remain viable producers of AChE for any period of time involve enormous expense such that the cost of producing the substantial quantities of AChE required for the prophylactic treatment against AChE inhibitors would be prohibitive. While techniques such as hybridization offer a possible means for "immortalizing" AChE-producing cells, the

amount of AChE produced by any given hybrid cell would likely be so small as to not significantly decrease the high cost (per unit product produced) of culturing such cells.

According to the present invention, the high cost, per unit product, of producing AChE by culturing cells which synthesize and secrete AChE is significantly reduced by isolating from a cell population those cells which produce AChE at maximum rates (i.e., cells which produce the greatest number of molecules of AChE per unit time), culturing such cells *in vitro* and recovering the AChE being produced and secreted by the culturing cells. Since the amount of AChE produced by such cells is maximally high, the cost of the overall culture and product removal process for a given amount of AChE produced is lower than heretofore achievable and makes economically feasible the production of the significant quantities of AChE required for administration to subjects as a preventitive measure against exposure to AChE inhibitors.

Any given cell line or population which produces and secretes AChE is heterogeneous with respect to the capability of individual cells within the population for producing particular quantities of AChE. This is true even for hybridoma cell lines grown up from a single hybrid cell. A number of cells within the population may be producing little or no AChE while a number of other cells may be producing a maximum amount of AChE per unit time. Culture of the entire cell population, including those cells making little or no AChE, is wasteful of culture medium, etc., but is tolerated since the isolation from the population, for culture, of only those cells producing AChE at a significant rate either is impossible or itself is so expensive as to offset any cost advantages to be gained in the culturing process.

The present invention relies upon a means for rapidly and inexpensively isolating from large populations of cells just those cells synthesizing and secreting AChE at a maximum rate, and thereafter culturing such cells *in vitro* and recovering the AChE secreted thereby. An understanding of the particular reason(s) why such cells produce AChE at a maximum rate is not *per se* required since the isolation methods depend upon the amount or rate of AChE produced by a given cell in comparisom to other cells in the population rather than upon the means by which such increased production occurs, be it by virtue of an even-present natural capability of the cell, some mutation of the cell or the phenomenon of gene amplification. However, the isolation techniques are deliberately designed such that if, for example, gene amplification is responsible for the heightened production of AChE, the conditions under which the isolation is carried out are those which might possibly result in an increase in the frequency of the gene amplification phenomenon.

The high-producing AChE cells isolated according to the present invention may be hybrid cells or unhybridized AChE-producing cells (which can, if desired, then be hybridized). The isolated cells may then be cultured *in vitro* in any suitable culture medium, such as standard tissue culture medium. In the preferred method of the invention, the culture medium for the cells is flowing lymph, which may be continuously derived from a living mammal, so as to closely simulate the conditions under which cells exist *in vivo*. See, e.g., my U.S. Patent Nos. 4,064,006 and 4,189,470.

Regardless of the culture medium or method employed, an additional feature of the present invention involves the periodic treatment of the culturing cells to isolate therefrom, for return to the culture unit, those cells

which still are producing AChE at an economically acceptable rate and to remove from the culture system those cells which, while originally isolated as high producers, have with time become unacceptably slow AChE producers or, indeed, have stopped producing AChE entirely.

Reference is now made, by way of example, to the accompanying drawings.

FIGS. 1A through 1E, 2A through 2C and 3A through 3C, schematically illustrate the isolation of a desired cell, i.e., one making AChE at a high rate, from cells either not making AChE or making AChE at a low rate.

A number of procedures may be employed for the isolation from a cell population of those cells synthesizing and secreting AChE at a high rate.

In the preferred method according to this invention, illustrated by FIGS. 1A through 1E, the individual cells of a cell population (cells 1 through 4) are encapsulated within a semi-permeable layer 10 along with a material capable of capturing and immobilizing within the capsule all AChE (if any) secreted by the cell over a given period of time (represented by dots in the figures). The capturing material is specific to AChE in the sense of being unable to capture and immobilize any other products which may be secreted by the cells. Preferred capturing and immbolizing materials are the known acetylcholine analogs which, like acetylcholine, specifically bind to AChE but which, unlike acetylcholine, are not hydrolyzed (or are only very slowly hydrolyzed) in the presence of AChE. Other suitable capturing and immobilizing materials for AChE include specific antibodies to any particular

determinant of the AChE molecule.

The capturing and immobilizing material *per se* is sized, and the permeability of the semi-permeable encapsulating material is chosen, such that the capturing material cannot escape from the capsule. The capturing material is caused to be present in the capsule in excess to insure that all AChE secreted by the cell over a given period of time is captured. Immobilization comes about by virtue of the binding between the AChE and the capturing material and the fact that the bound complex is too large to permeate through the semi-permeable encapsulating material.

The entire population of encapsulated cells is suspended in any medium in which the cells (and their ability to synthesize and secrete AChE) remain viable for the duration of the isolation process, and which is of a density such that all the encapsulated cells, *ab initio*, remain in relatively uniform, fixed vertical position within the suspension.

After a given predetermined period of time sufficient to allow any cells capable of so doing to synthesize and secrete AChE, the condition of the cell population is represented by FIG. 1B, wherein, in those capsules containing a cell secreting AChE (i.e., the capsules containing cells 1 and 3) the AChE has been captured and immobilized within the capsule. Thereafter, there is added to the entire cell population a product (represented by _____ in FIG. 1C) which is capable of specifically binding only to AChE or to the complex of AChE and capture material. For example, this product can be an acetylcholine analog or an antibody to AChE which binds to AChE at a site or determinant thereon other than the site or determinant at which the capture material is bound. Bound to the added product (e.g.,

via covalent binding) is an enzyme ( E ). The product-enzyme complex is sized such that it is capable of permeating the capsule through the semi-permeable encapsulating material.

Owing to the specific affinity of the added product for AChE or the AChE/capture material complex, the added product will become bound only in those capsules where AChE is present, i.e., only in those capsules containing a cell synthesizing and secreting AChE. The size of the resultant complex is such that it can no longer escape from the capsule through the semi-permeable encapsulating material. Accordingly, after treating the system to remove unbound added product and enzyme (e.g., by washing or through use of immunosorbents or other affinity materials), the result is that enzyme is present only in those capsules containing cells secreting AChE (see FIG. 1D). More importantly, the absolute amount of enzyme present in any such capsule is a direct function of the amount of AChE present in the capsule, i.e., the amount of AChE secreted by the cell over a given period of time. Accordingly, those capsules containing cells producing and secreting AChE at a high rate (molecules of AChE per unit time) will contain more enzyme than those capsules containing cells producing AChE at a lower rate (compare the capsules containing cell numbers 1 and 3).

The entire population is then treated with a substrate which is acted upon by the enzyme so as to bring out a change (or fail to bring about a change) only in those capsules containing the enzyme, i.e., only in those capsules containing cells secreting AChE. Moreover, the degree or magnitude or intensity of the change is a function of the amount of enzyme present and, thus, in turn, a function of the amount of AChE secreted by the cell over a given period of time.

The choice of enzyme and substrate is predetermined, of course, and may be any combination capable of bringing about a visible or measurable change in the system. By way of example, the substrate may be a colorless material which is converted to a colored material in the presence of the enzyme. Those capsules containing color are, therefore, those capsules containing a cell secreting AChE, with the intensity of the color being a measure or indicator of the rate at which such cell produces AChE.

Color differences, however, may be difficult to accurately distinguish and/or may require utilization of measuring equipment having inherent limitations as to the number of cells which can be measured or viewed at a single time. As will become more apparent hereinafter, the economics of the AChE production according to the present invention and the ability to find cells producing AChE at a high rate are greatly enhanced by the ability to subject large populations of cells to isolation techniques at a single time and to achieve the desired isolation rapidly. Accordingly, the preferred isolation technique of the present invention utilizes as a substrate a soluble material which, in the presence of enzyme, is converted to an insoluble precipitate. The formation of insoluble precipitate in those capsules containing the enzyme, i.e., in those capsules containing a cell secreting AChE, causes the capsules to increase in mass and physically "separate" (i.e., sink) from other capsules not containing the enzyme. Since the rate of conversion of the soluble material to an insoluble precipitate and the absolute amount of precipitate formed in any given capsule is a function of the amount of enzyme present therein, either the time for descent of a capsule to begin, or the rate of descent of a capsule, or the depth to which a capsule descends, can be used as a visible relative measure of

the amount of AChE present in the capsule and, thus, the AChE production rate of the cell within the capsule (see FIG. 1E).

In a similar technique, the enzyme and substrate system can be chosen such that the enzyme converts the substrate into gaseous products which will cause the capsules in which enzyme is present to levitate in the medium and separate from other cells in the population. Again, the time for levitation of a capsule to begin, the rate of capsule ascent or the level to which a capsules rises can be used as a measure of the relative amount of AChE present in the capsule.

After this isolation procedure, those capsules in which cells producing AChE at the highest rate are contained (as determined in the manners set forth above) can then be removed from the system, treated to remove the encapsulating semi-permeable material and the cells contained therein cultured in a suitable medium to propagate the growth of the cells and their production and secretion of AChE. AChE can then be removed from the culture medium at regular intervals after a suitable number of cells have grown up through division and sub-division.

In the preferred practice of this invention, however, cells found by the described isolation techjniques to be producing AChE at high rates are re-subjected to isolation procedures so as to still further cull from this population the cells producing AChE at the very highest rates. Thus, the cells collected in the first isolation can be cultured in a suitable manner to provide a new population of cells which are then encapsulated and subjected to isolation techniques as earlier described. In this and any subsequent "re-isolation," the same or different capture materials,

products, enzymes, etc. utilized in the first isolation may be employed. Indeed, since the cells subjected to re-isolation procedures necessary are cells known to be producing only AChE, it may be possible in these re-isolations to employ capture material and added product capable of binding to AChe but which do not necessarily have to be highly specific to AChE alone, the assumption being that no products other than AChE, which could bind to the capture material and added product, will be produced and secreted by these cells.

Enzyme-based isolation techniques other than those described earlier may be employed according to this invention, for example, where the substrate is a toxic material which, in the presence of enzyme, is converted to a non-toxic material. Cells within capsules containing enzymes (i.e., AChE-secreting cells) will therefore survive such an environment and, after normal division and subdivision of the cells, the capsules will eventually become heavier than capsules in which cell death has occurred (i.e., capsules not containing AChE-secreting cells or producing quantities of AChE too low to have re presencd in the capsule of sufficient enzyme to counter the toxic environment). Repeated isolations, under increasing dosages of the toxic material, will eventually isolate only those cells producing AChE at the highest possible rates.

In a further isolation technique according to the present invention, cells producing AChE at a high rate can be isolated from all other cells in a cell population in a procedure illustrated by FIGS. 2A through 2C. In this procedure, the cells are arranged in a medium which permits the cells to remain viable during the isolation period and which limits the diffusivity and mobility of products secreted by the cells. As illustrated in FIG. 2A, AChE secreted by any

cell in the population will remain in the immediate vicinity of the cell secreting it; cells not secreting AChE will have no AChE in their immediate vicinity. After a suitable amount of time has elapsed for secretion of AChE, there is added to the entire system a material which specifically binds to AChE, such as an AChE inhibitor, an antibody to AChE or a slowly-hydrolyzed acetylcholine analog. The material has bound to it a poison, such as ricin, which is toxic to cells only if it penetrates the cell (see FIG. 2B). As shown in FIG. 2C, cells secreting large quantity of AChE will be protected from the poison since the AChE remains in the immediate vicinity of the cell and intercepts and prevents the material to which the poison is bound to penetrate the cell. Cells producing no AChE or only small quantities of AChE cannot similarly tie up the material to which the poison is bound and the poison therefore enters and kills such cells. The remaining living cells in the population, therefore, are those producing AChE at a high rate and are then recovered from the population. Repeat isolations using increasing concentrations of AChE-binding material and toxin will eventually isolate only those cells secreting AChE at a very high rate.

Obviously, this same technique can be applied to cells individually encapsulated in semi-permeable material. In this case, beginning with a suspension of such capsules, the capsules containing surviving cells (i.e., cells secreting AChE at high rates) will eventually descend owing to the increased weight brought about by division and sub-division of the living cells therein.

In yet another isolation technique, illustrated in FIGS. 3A through 3C, the cells subjected to the isolation procedure are cells which mandatorily require choline in order to remain viable. Such cells can be found existing

in nature as such or can be induced to possess this property through the use of drugs.

The cells are then suspended in a medium deficient in choline and which limits the diffusivity and mobility of products secreted by the cells. Thus, after a given period of time (see FIG. 3A), cells which secrete AChE will have AChE in their immediate vicinity and in the cells *per se*. Thereafter, acetylcholine (or an acetylcholine analog), which is hydrolyzable in the presence of AChE to form choline, is added to the entire system (see FIG. 3B). In the vicinity of those cells secreting AChE, the acetylcholine will be converted to choline and such cells will, therefore, be able to survive in the otherwise choline-deficient medium (see FIG. 3C). All other cells eventually will die, and the living cells, now known to be AChE producers, can be isolated therefrom.

Isolation of only those cells making AChE at a high rate can be facilitated by first treating the cell population with a predetermined dose of an AChE inhibitor. In those cells making AChE at a low rate, the inhibitors will inactivate or tie up all the AChE and thereby render it unavailable for converting the later-added acetylcholine to the choline required by the cells for life. Only in those cells making AChE at a high rate will sufficient AChE remain present after addition of the inhibitor to convert enough acetylcholine to choline to permit the cells to live.

The foregoing isolation technique also can be practiced with a semi-permeable encapsulating material, whereby the capsules containing dividing and sub-dividing living cells eventually self-separate from all other cells owing to their increased weight.

With respect to those isolation techniques described herein which employ encapsulated cells, a number of techniques are available for forming such capsules or beads containing the individual cells of the cell population and any other materials (e.g., the chosen capture material required in certain embodiments). Generally, the techniques involve formation of a suspension of the cell population (and other materials) in an appropriate gel-forming medium such as alginate or agarose. The suspension is constantly stirred to insure homogeneity and, e.g., a needle-like device is employed to draw off very small quantities thereof and deposit them as droplets into a medium which causes the droplets to form coherent beads. Thus, for example, where alginate is the gel-forming medium, the droplets may be deposited in a calcium ion-containing solution (e.g., $CaCl_2$) to cause gellation of the alginate. Where agarose is the gel-forming medium, the droplets may be deposited in an oil solution maintained at a temperature sufficiently low to cause the agarose to gell. A mixture of alginate and agarose may also be employed as the gel-forming medium, in which case the droplets are deposited into a calcium-containing solution maintained at low temperature.

It is also possible to employ droplet forming and depositing devices which are fed, from separate metered inlet sources, with cells (and other materials) and the gel-forming medium, after which a droplet of the now mixed cells and medium is deposited into a solution (e.g., calcium-containing or cold oil) which causes gellation. Beads or capsules may also be formed by emulsifying cells and gel-forming medium to form small, discrete droplets containing cells and the gel medium, after which the droplets are subjected to conditions which cause their gelation into discrete spherical beads.

Other means for forming beads containing individual cells of the cell population incude means whereby a perforated cylinder is used to contain a suspension of cells and gel-forming medium. The cylinder is itself placed in a medium appropriate for gelling whatever gel-forming material is employed, and is rotated therein so as to spin-off, through the perforations, small discrete droplets of gel-forming medium and cells. These droplets immediately encounter the medium which causes gelation of the gel-forming material (e.g., cold oil, calcium-containing solution) and results in the formation of coherent beads.

In each of the bead-forming mechanisms, the concentration of cells, the concentration of gel-forming medium and the size of droplets formed are chosen such that each droplet (and, hence, each bead ultimately formed therefrom) contains one cell and of the cell population and, where appropriate, the specified number of other materials such as an appropriate capture material.

The gelled beads or microspheres formed according to any of the foregoing techniques may be used <u>per se</u> as the beads or capsules in the various isolation methods previously described. Generally, however, it is preferred to surround each gelled microsphere with a semi-permeable material and then dissolve the gelled material of the microsphere so as to form a bead, containing the components present in the gelled microshpere, but now constituted of the semi-permeable material as the bead material. Thus, for example, alginate microspheres can be surrounded by a layer of polylysine (via electrostatic attraction) and the thus-formed beads subjected to a medium which removes calcium ions from the microshperes (e.g., a solution of the calcium sequestering agent or a calcium-free solution).

This causes the microspheres to liquify and the result is a bead consisting of polylysine encapsulating the cell and other components present in the originally formed microsphere. The same technique can be employed where the gelled microspheres were formed from agarose, wherein the agarose microspheres, surrounded by a semi-permeable material such as polylysine, are subjected to, e.g., hot oil or other fluid so as to dissolve the agarose gel and result in a polylysine bead. (Since polylysine has little or no electrostatic attraction toward agarose, it may be desirable to utilize a small amount of alginate in forming the predominantly agarose microspheres, sufficient to cause the polylysine to be electrostatically attracted to, and surround, the microsphere; the alginate portion of the microsphere in the resultant bead can then be dissolved as earlier described).

The beads or capsules may also be prepared by metering appropriate amounts of cell suspension, oil and water into a permeable hollow fiber and progressively removing water from the so formed capsules as they transverse the length of the fiber.

Since a number of the isolating techniques described depend upon establishing weight differentials between capsules or beads containing desired cells (i.e., cells producing AChE at a high rate) and capsules or beads not containing such desired cells, it is desirable to arrange (so far as is possible) that the weight differential comes about entirely or predominantly by virtue of the differences in the cells, and the reactions set in motion by virtue of the different AChE production rates of the cells, rather than any other extraneous factors. Thus, for example, the mechanism of bead or capsule formation is such that, statistically, a single cell from the cell population and, where

applicable, quantities of one or more other materials (e.g., capture material) are contained within each bead. However, it may turn out that certain beads contain more or less cells or capture material than other beads and, moreover, may differ in overall size, weight and density. When this full distribution of beads is employed in one of the earlier-described weight of density-dependent isolation techniques, the weight or density differentials caused to occur (e.g., via precipitate formation) between beads containing desired cells and beads containing undesired cells may be on the same order of magnitude as some of the weight or density differentials inherent in the beginning beads _per se_. As such, a number of beads may be incorrectly isolated simply because they were heavier or lighter than other beads to begin with.

This possible difficulty can be overcome by arranging that the intentionally caused weight or density differentials are very much larger than any inherent differentials in the beginning beads. A more preferred solution, however, is to subject the formed beads, prior to use in an isolation technique, to a means whereby beads of closely similar weight and density aggregate. Thus, for example, all the beads can be added to a vertical column containing a fluid of specified viscosity and density such that beads of the same or closely similar weight and density will collect together at various levels in the fluid. For use in an isolation technique, only beads from the same fluid level are employed, thereby insuring that weight and density differentials noted in the isolation process are the result of intentionally caused conditions rather than inherent dissimilarities in the starting bead population.

Cells producing AChE at a high rate, isolated from cell

populations according to the foregoing techniques, are thereafter cultured in a suitable medium to propagate their growth and their production of AChE. The cells subjected to culture may be the isolated AChE-producing cells *per se* or (if these cells were not hybrid cells to begin with) hybrid cells prepared therefrom according to known hybridization techniques. See, e.g., U.S. Patent Nos. 4,172,124 and 4,196,265 and published European patent application Specification No. 0028902 (published May 20, 1981)). Through use of the isolation techniques described herein, it will be noted that the cell population resulting from such hybridization can be rapidly analyzed to recover therefrom just those hybrids making AChE at any or some high rate. Accordingly, the parent cell used to impart immortality to the hybrid cell (e.g., a myeloma cell) need not, as is customary in the art, be a particular mutated cell possessing some characteristic which brings about its eventual death unless it becomes hybridized to a normal (e.g., AChE-producing) cells. (For example, the art customarily employs mutant myeloma cells deficient in hypoxanthine phosphoribosyltransferase (HPRT); the growth of such cells is inhibited when the cell population resulting from hybridization is suspended in selective hypoxanthine-aminopterin-thymidine (HAT) medium). Using the isolation techniques of the present invention, hybrid cells producing AChE can simply be selectively isolated from all other cells in the population resulting from the hybridization process.

As earlier noted, the AChE-producing cells may be cultured in any means and medium in which the cells are viable and from which AChE secreted by the cells can be recovered. AChE recovery methods can be chosen from a variety of available techniques employing chemically specific or biologically specific sorbents therefor, such as acetylcholine analogs or antibodies to AChE,

from which the AChE can be decomplexed.

The preferred method for culturing the cells involves the use of flowing lymph as the culture fluid (or as a predominant part of the culture fluid), preferably lymph obtained "on-line" from a mammal such as a cow or a sheep. See, e.g., U.S. Patent Nos. 4,064,006 and 4,189,470. In a preferred lymph culture process, the cells to be cultured are physically separated from the flowing lymph by a semi-permeable membrane (e.g., in the form of a flat sheet or in the form of a hollow fiber with the cells on either the inside or outside of the fiber), across which components of the lymph contact the cells to provide nutrition, necessary regulatory or helper molecules, etc. and waste from the cells is carried away by the lymph. The lymph typically is rendered cell-free before being used in the culturing process, but the semi-permeable membrane per se may be used to prevent cells in the lymph from intermingling with the culturing cells. Means are provided to supply oxygen to the culturing cells either through oxygen-supplying or liberating materials in the lymph stream or in contact with the culturing cells or through use of lungs through which the culturing cells are continuously or periodically pulsed.

Preferably, the semi-permeable barrier between the flowing lymph and the culturing cells is sized such that AChE produced by the culturing cells remains on the "cell-side" of the membrane barrier. The AChE can be removed continuously or periodically from the cell-side of the culture unit either by (a) removal of all or part of the cells and fluid from the cell-side of the culture chamber and either (i) contacting the cells and fluid with a sorbent for AChE or (ii) first separating the cells and fluid and passing only the fluid through an AChE sorbent; or (b) having AChE affinity material

directly in with the culturing cells on the cell-side of the culture unit and removing the affinity material at regular intervals. In cases where cells are removed from culture for AChE recovery, the cells may thereafter be returned to the culture unit for further AChE production.

The flowing lymph, preferably obtained on-line from a mammal, can be treated to add or subtract molecules therefrom to enhance the simulated in vivo environment and, after use in culture, may be returned to the mammal.

As discussed earlier in this application, the preferred method for culturing cells producing AChE at a high rate includes a method for periodically removing the cells from culture and, by subjecting the cells to any of the described isolation techniques, isolating (for continued culture) only those cells still producing AChE at an economically acceptable high rate. For continuous or semi-continuous culture techniques, this periodic screening of the cells can best be accomplished through use of two or more culture units from and to which cells are alternately removed and supplied in a sequence which submits the cells to isolation/screening intermediate the culture units.

CLAIMS:

1. A method for isolating living mammalian cells secreting acetylcholinesterase, characterised by the steps of:

(a) obtaining a population of cells containing cells secreting acetylcholinesterase at varying rates;

(b) encapsulating the individual cells of said population within a semi-permeable material along with a capture material capable of specifically binding to acetylcholinesterase, said semi-permeable material being sized such that neither the capture material nor capture material bound to acetylcholinesterase can permeate therethrough;

(c) allowing to elapse a predetermined period of time sufficient for acetylcholinesterase-producing cells to secrete acetylcholinesterase to bind to said capture material;

(d) adding to the entire cell population a product having an enzyme affixed thereto, the product being permeable through said semi-permeable material and capable of binding specifically to acetylcholinesterase or the acetylcholinesterase lowered to the capture material, said product having affixed thereto an enzyme, said product and enzyme becoming immobilized only within the semi-permeable encapsulating material in which a cell producing acetylcholinesterase is contained, the amount of enzyme

and product so immobilized being directly proportional to the amount of acetylcholinesterase secreted by the cell in a given period of time;

(e) removing from the cell population any non-immobilized product and enzyme;

(f) adding to the entire cell population a substrate capable of being acted upon by said enzyme to bring about a measurable or identifiable change only at those encapsulated cells where enzyme is immobilized, the magnitude of such change being directly proportional to the quantity of immobilized enzyme present in a capsule; and

(g) recovering those encapsulated cells in which a predetermined degree of identifiable change has occurred.

2. A method according to claim 1 for isolating living mammalian cells secreting acetylcholinesterase characterised in that the capture material of step (b) is chosen from the group consisting of acetylcholine analogs which specifically bind to acetylcholinesterase and which generally remain in the ester group configuration in the presence of acetylcholinesterase, and specific antibodies to any particular determinant of the acetylcholinesterase molecule.

3. A method according to claim 1 or 2 for isolating living mammalian cells secreting acetylcholinesterase characterised in that the capture material of step (b) is in an excess amount such that

the acetylcholinesterase secreted by the cell over a given period of time is specifically bound by the capture material.

4. A method according to claim 1, 2 or 3 for isolating living mammalian cells secreting acetylcholinesterase which is further characterised by the step of suspending the encapsulated individual cells of the population in a medium in which the cells remain viable and which is of a density such that the encapsulated cells remain in relatively uniform, fixed vertical position within the suspension for the duration of the isolation process.

5. A method according to any one of the preceding claims for isolating living mammalian cells secreting acetylcholinesterase characterised in that the complex of acetylcholinesterase and capture material of step (d) is too large to permeate through the semi-permeable encapsulating material.

6. A method according to any one of the preceding claims for isolating living mammalian cells secreting acetylcholinesterase characterised in that the substrate of step (f) is a soluble material which is converted to an insoluble precipitate in the presence of the enzyme such that the insoluble precipitate is formed at those encapsulated cells containing the enzyme and acetylcholinesterase which causes those encapsulated cells to increase in mass and physically separate from other encapsulated cells not containing the enzyme and acetylcholinesterase.

7. A method according to any one of claims 1 to 5 for isolating living mammalian cells characterised in that the substrate of step (f) is converted by the enzyme into gaseous products such that the encapsulated cells at which the enzyme is present levitate in the medium and separate from other cells in the population.

8. A method according to any one of claims 1 to 5 for isolating living mammalian cells secreting acetylcholinesterase characterised in that the substrate of step (f) is a material toxic to the cells of the population which, in the presence of the enzyme is converted to a non-toxic material such that the encapsulated cells containing a sufficient quantity of enzyme will survive, divide and eventually cause those encapsulated cells to become heavier than encapsulated cells in which cells have been destroyed.

9. A method according to any one of the preceding claims for isolating living mammalian cells secreting acetylcholinesterase which is further characterised by the step of repeating the steps for isolating for resubjecting to isolation the encapsulated cells in which a predetermined degree of identifiable change has occurred so as to further cull the cells producing acetylcholinesterase at the very highest rates.

10. A method according to claim 1 characterised in that said encapsulated cells produced in step (b) are suspended in a medium in which the cells therein are viable and which maintains such encapsulated cells in a relatively fixed position within said medium, and

wherein said substrate is a soluble material which is converted by said enzyme into an insoluble precipitate, whereby the precipitate formation at encapsulated cells containing enzyme causes the encapsulated cells to become heavier and separate from all other encapsulated in the suspension at a rate and to a degree proportional to the amount of enzyme present at the encapsulated cells.

11. A method for isolating living mammalian cells secreting acetylcholinesterase, characterised by the steps of:

(a) obtaining a population of cells containing cells producing and secreting acetylcholinesterase at varying rates;

(b) arranging the cells of the population in a medium which permits the cells to remain viable while the cells secreting acetylcholinesterase are being isolated and which limits the diffusivity and mobility of products secreted by the cells such that acetylcholinesterase secreted by any cells in the population will remain in the immediate vicinity of the cell secreting it and cells not secreting acetylcholinesterase will have no acetylcholinesterase in their immediate vicinity;

(c) allowing to elapse a predetermined period of time for cells producing acetylcholinesterase to secrete acetylcholinesterase; and

(d) adding to the entire cell population a material which specifically binds to acetylcholinesterase and has bound to it a poison which is toxic to the cells of the population only if it penetrates the cells such that cells secreting a large quantity of acetylcholinesterase will be protected from the poison and cells producing little or no acetylcholinesterase cannot similarly be protected and will be destroyed.

12. A method according to claim 11 for isolating living mammalian cells secreting acetylcholinesterase characterised in that the material which specifically binds to acetylcholinesterase of step (d) is chosen from the group consisting of an acetylcholine analog, an acetylcholinesterase inhibitor, and an antibody to acetylcholinesterase and in which the poison is ricin.

13. A method according to claim 11 or 12 for isolating living mammalian cells secreting acetylcholinesterase which is further characterised by the step of repeated isolations using increasing concentrations of the material which specifically binds to acetylcholinesterase and its bound poison of step (d) such that only those cells secreting acetylcholinesterase at a very high rate will be isolated.

14. A method according to any one of claims 11, 12 and 13 for isolating living mammalian cells secreting acetylcholinesterase which is further characterised by the step of individually encapsulating the cells of the population containing surviving cells

such that the capsules containing surviving cells will eventually descend due to the increased weight brought about by the division of the living cells therein.

15. A method for isolating living mammalian cells secreting acetylcholinesterase which is characterised by the steps of:

(a) obtaining a population of cells containing cells producing and secreting acetylcholinesterase and which require choline in order to remain viable;

(b) suspending the cells in a medium deficient in choline which limits the diffusivity and mobility of products secreted by the cells;

(c) allowing to elapse a predetermined period of time sufficient for cells producing acetylcholinesterase to produce and secrete acetylcholinesterase; and

(d) adding to the entire system a substance which is hydrolyzable in the presence of acetylcholinesterase to form choline such that the substance is converted to choline in the vicinity of those cells secreting acetylcholinesterase to the extent necessary for their survival in an otherwise choline free medium.

16. A method for isolating living mammalian cells secreting acetylcholinesterase according to claim 15, which is further characterised by the step of treating

the cell population with a predetermined dose of an acetylcholine inhibitor such that only those cells making acetylcholinesterase at a predetermined rate will survive after addition of the inhibitor and the substance.

17. A method according to claim 15 or 16 for isolating living mammalian cells secreting acetylcholinesterase characterised in that the substance is acetylcholine analog.

18. A method according to claim 15, 16 or 17 for isolating living mammalian cells secreting acetylcholinesterase characterised in that the cells are individually encapsulated in a semi-permeable encapsulating material, such that the capsules containing dividing living cells will eventually separate from other cells due to their increased weight.

19. A method for a preventative treatment against the effects of the cholinergic nervous system on a subject of an invading acetylcholinesterase inhibitor characterised by the steps of:

(a) administering acetylcholinesterase to the subject;

(b) permitting the acetylcholinesterase to pass into the circulatory system of the subject, the amount of acetylcholinesterase administered to the subject

(i) being an amount sufficient to establish a concentration thereof in the circulatory system of the subject which is substantially below the concentration of acetylcholinesterase present on or near cells involved in the cholinergic nervous system; and

(ii) being at least equal to the amount necessary to establish a concentration in the circulatory system of the subject to intercept and tie up the invading acetylcholinesterase inhibitor before the inhibitor can act upon the acetylcholinesterase involved in the functions of the cholinergic nervous system of the subject.

Fig. 1A.

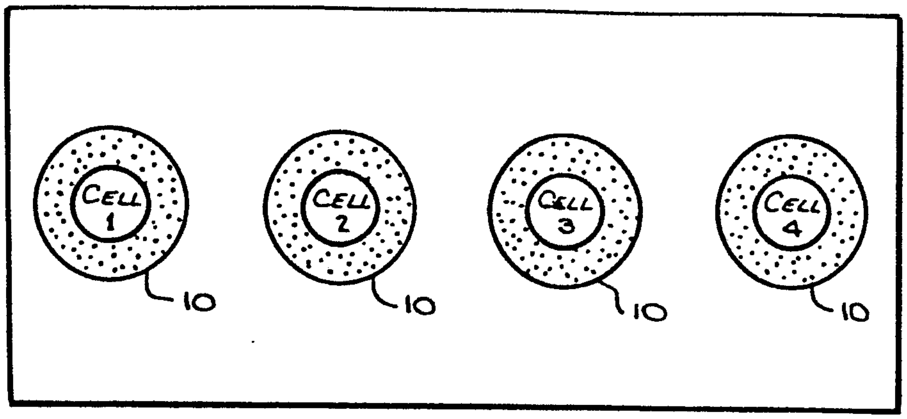


Fig. 1B.

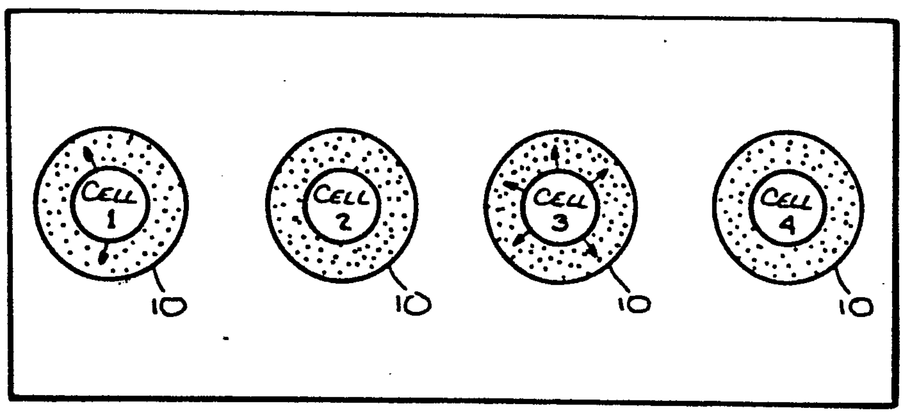


Fig. 1C.

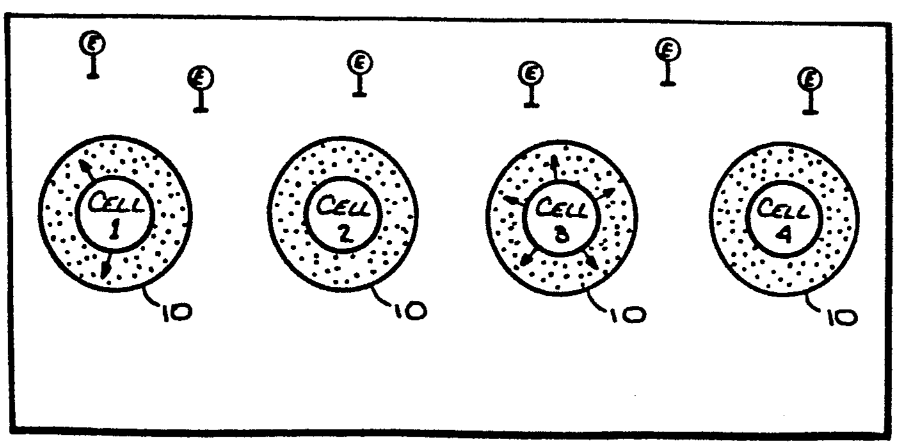

0114756

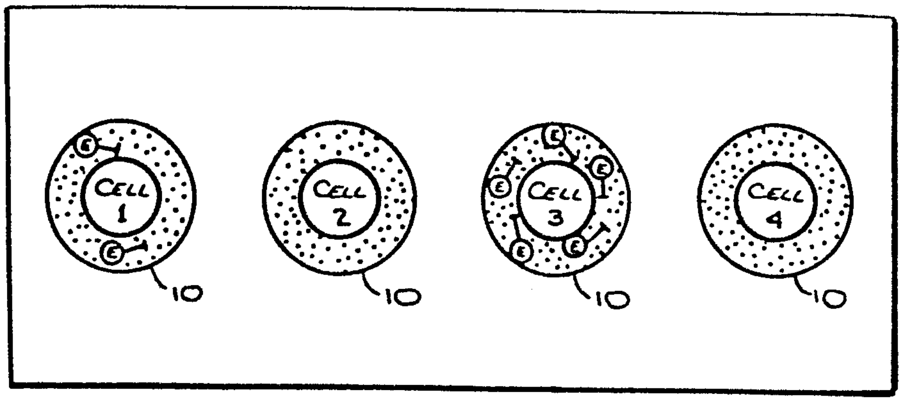


Fig. 1D.

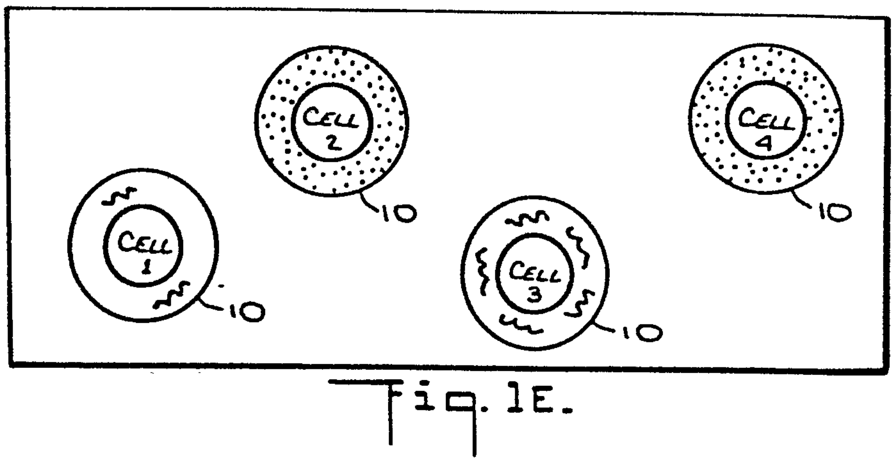


Fig. 1E.

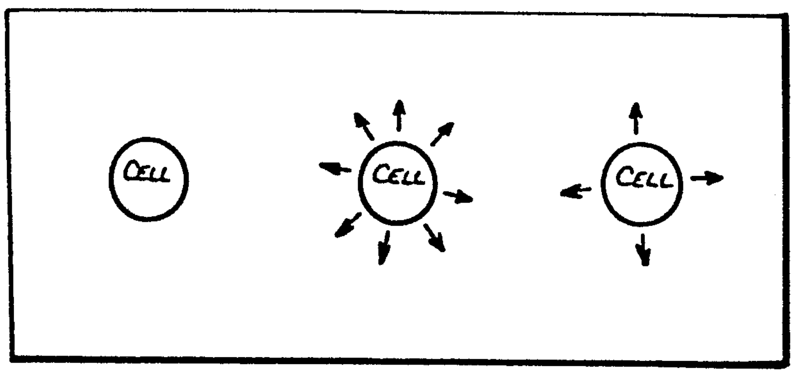


Fig. 2A.

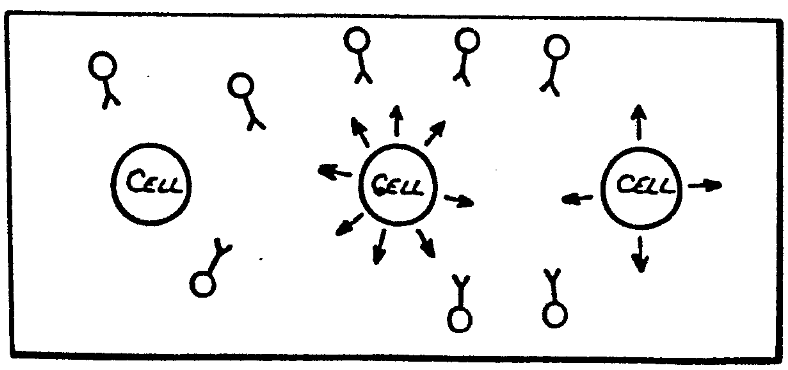


Fig. 2B.

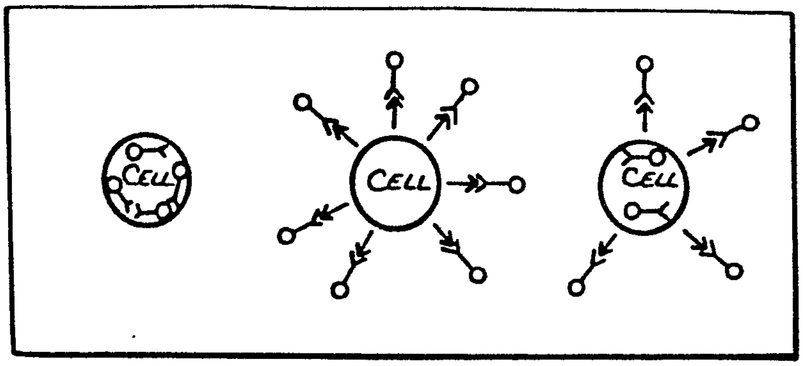


Fig. 2C.

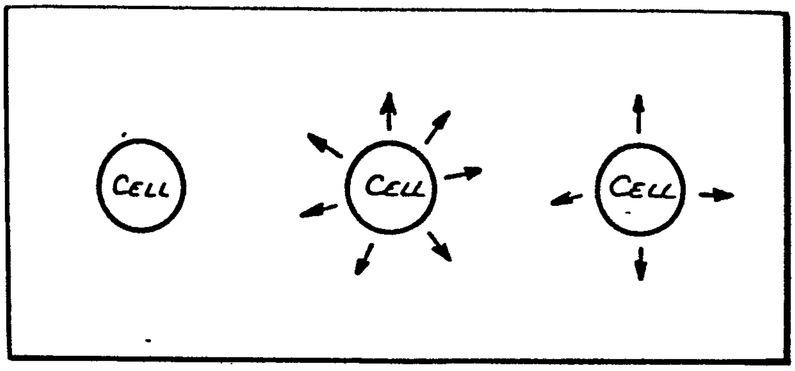


Fig. 3A.

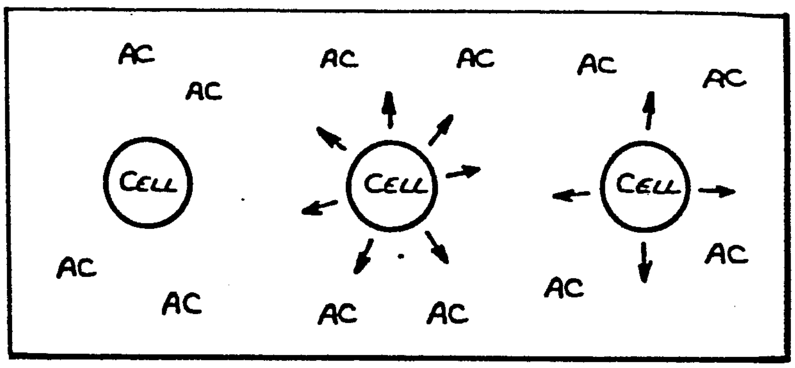


Fig. 3B.

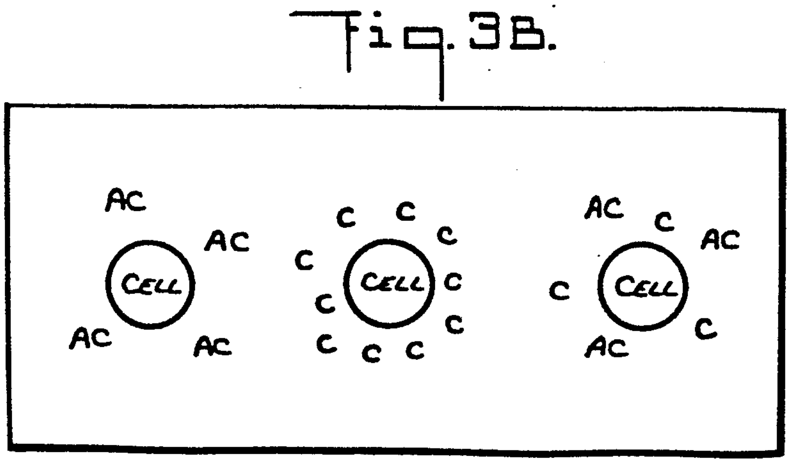


Fig. 3C.